# EUROPÄISCHE PATENTANMELDUNG

(11) **EP 1 072 225 A2**
(43) Veröffentlichungstag der Anmeldung: **31.01.2001**
(21) Anmeldenummer: 00115668.6
(22) Anmeldetag: 20.07.2000
(51) Int. Cl.: A61B 17/122

(54) **Chirurgischer Nahtclip**

(30) Priorität: 29.07.1999 DE 19935637
(71) Anmelder: Aesculap AG & Co. KG, 78532 Tuttlingen (DE)
(72) Erfinder: Dworschak, Manfred, 78589 Dürbheim (DE); Weisshaupt, Dieter, 78194 Immendingen (DE)
(74) Vertreter: Böhme, Ulrich, Dr. Dipl.-Phys.

(57) **Zusammenfassung**

Um bei einem chirurgischen Nahtclip mit zwei gegeneinander verschwenkbaren Klemmarmen, die im gegeneinandergeklemmten Zustand mindestens einen Nahtfaden zwischen sich festlegen, eine sichere und verletzungsfreie Festlegung von Nahtgut unterschiedlichen Durchmessers zu ermöglichen, wird vorgeschlagen, daß die Klemmarme über einen verformbaren und nach Verformung seine Form beibehaltenden Steg miteinander verbunden sind.

## Beschreibung

Die Erfindung betrifft einen chirurgischen Nahtclip mit zwei gegeneinander verschwenkbaren Klemmarmen, die im gegeneinandergeklemmten Zustand mindestens einen Nahtfaden zwischen sich festlegen.

Bei der Verbindung chirurgischer Nahtfäden sind Chirurgen üblicherweise so vorgegangen, daß die Enden dieser Nahtfäden miteinander verknotet werden. Dies ist insbesondere bei endoskopischer Operationsweise außerordentlich kompliziert und zeitaufwendig, und daher ist es bekannt, Verknotungen des Nahtmaterials dadurch zu ersetzen, daß an die Enden des Nahtmaterials chirurgische Nahtclips angelegt werden, deren Klemmarme gegeneinandergedrückt werden und zwischen sich die Enden des Nahtmaterials aufnehmen. Derartige chirurgische Nahtclips sind beispielsweise bekannt aus der EP 0 519 703 B1, der EP 0 519 704 B1 oder der US-A-5,462,558. All diesen Clips ist gemeinsam, daß die beiden Klemmarme über einen scharnierartig ausgebildeten Steg reversibel gegeneinander verschwenkbar sind und daß sie in der geschlossenen Position dadurch festgelegt werden, daß Vorsprünge an einem Klemmarm Rücksprünge am anderen Klemmarm rastend hintergreifen. In der Praxis hat sich herausgestellt, daß diese chirurgischen Nahtclips zwar bei bestimmten Fadenstärken des Nahtmaterials einsetzbar sind, daß sie aber nicht universell geeignet sind für unterschiedliche Fadenstärken, da der gegenseitige Abstand der Klemmarme allein durch die Rastverbindung bestimmt wird und immer gleich ist, so daß bei sehr dünnen Fäden die Gefahr eines Abrutschens besteht, bei sehr dicken Fäden hingegen die Gefahr einer Verletzung des sehr empfindlichen Nahtmaterials durch zu starke Klemmung.

Es ist Aufgabe der Erfindung, einen chirurgischen Nahtclip der gattungsgemäßen Art so auszubilden, daß der Operateur diesen an Nahtmaterial unterschiedlicher Fadenstärke anlegen und dabei so gestalten kann, daß er in jedem Falle die Enden des chirurgischen Nahtmaterials sicher und ohne übergroße Klemmung hält.

Diese Aufgabe wird bei einem chirurgischen Nahtclip der eingangs beschriebenen Art erfindungsgemäß dadurch gelöst, daß die Klemmarme über einen verformbaren und nach Verformung seine Form beibehaltenden Steg miteinander verbunden sind.

Die Klemmarme dieser Nahtclips werden also dadurch gegeneinandergespannt, daß der Nahtclip im Bereich des verformbaren Steges verbogen wird, diese Verformung wird beibehalten, so daß der Steg die Klemmarme elastisch gegeneinanderspannt. Der Operateur hat dabei die Möglichkeit, beim Zusammenbiegen der Klemmarme die Schließkraft so zu wählen, daß die Klemmarme unter bestimmter Spannung an den Fadenenden anliegen, und zwar unabhängig vom Durchmesser der jeweils verwendeten Fäden. Wenn die Clips einmal in dieser Weise zusammengebogen sind, behalten sie ihre durch Verformung erreichte Gestalt und halten somit die Fadenenden sicher und verletzungsfrei zwischen den Klemmarmen.

Obwohl es grundsätzlich möglich wäre, diese Nahtclips aus Kunststoff herzustellen, insbesondere aus einem resorbierbaren Kunststoff, ist es doch vorteilhaft, wenn der Steg aus Metall besteht, da gerade bei dieser Ausgestaltung aus Metall der Steg besonders hohe Klemmkräfte aufbringen kann.

Günstig ist es auch, wenn der Steg und die Klemmarme aus einem durchgehenden Bauteil aus Metall bestehen, wenn also der ganze Clip aus einem länglichen, metallischen Bauteil geformt ist.

Insbesondere können der Steg und gegebenenfalls die Klemmarme aus Titan oder aus einer Titanlegierung bestehen.

Es ist vorteilhaft, wenn der Steg V-förmig ausgebildet ist, so daß durch die spitzwinkelige Ausbildung ein Stegbereich vorgegeben ist, der sich beim Zusammenbiegen des Clips besonders stark verformt.

Insbesondere kann vorgesehen sein, daß die Klemmarme geradlinig ausgebildet sind und unter Ausbildung eines Winkels oder einer Abbiegung in den Steg übergehen. Man erhält dann einen Clip mit geradlinigen Klemmarmen und mit geradlinigen Stegabschnitten, die im Übergangsbereich zu den Klemmarmen und zwischen sich winkelige oder starke gekrümmte Bereiche aufweisen, so daß beim Zusammenbiegen dieser Clips diese winkeligen oder stark abgebogenen Bereiche bevorzugt verformt werden, im Bereich zwischen den Stegabschnitten durch eine Vergrößerung der Abbiegung, im Bereich zwischen den Klemmarmen und den Stegabschnitten dagegen durch eine Begradigung des Übergangsbereiches.

Bei einer besonders bevorzugten Ausführungsform ist vorgesehen, daß der Nahtclip zumindest im Bereich der Klemmarme von einem weichen Material umgeben ist. Weich bedeutet dabei ein Material, das gummiähnliche Eigenschaften aufweist und zu einer zuverlässigen Einbettung und Umgreifung der Fadenenden führt, so daß sich die Klemmarme unter Zwischenlage des weichen Materials an die zwischen sich eingeklemmten Fadenenden anschmiegen und diese dadurch sicher zwischen den Klemmarmen festlegen.

Dieses Material kann beispielsweise Silikon sein.

Bei einer ersten bevorzugten Ausführungsform ist vorgesehen, daß zumindest die Klemmarme und gegebenenfalls der gesamte Clip einen Tauchüberzug aus dem weichen Material tragen. Dieser wird dadurch aufgebracht, daß der Clip in einen flüssigen Vorrat des aufzubringenden weichen Materials eingetaucht wird und daß der dadurch auf dem Clip entstehende Überzug fest wird, beispielsweise durch Erkalten.

Gemäß einer anderen bevorzugten Ausführungsform ist vorgesehen, daß zumindest die Klemmarme und gegebenenfalls der gesamte Clip einen Spritzüberzug aus dem weichen Material tragen. Bei dieser Ausführungsform wird das weiche Material durch Aufspritzen auf die Klemmarme und gegebenenfalls auf den gesamten Clip aufgetragen.

Besonders bevorzugt ist eine Ausführungsform, bei der vorgesehen ist, daß das weiche Material die Form eines Schlauches aufweist, der zumindest über die Klemmarme und gegebenenfalls über den gesamten Clip gezogen ist.

Günstig ist es, wenn das weiche Material über die freien Enden der Klemmarme übersteht, so daß in diesem Bereich die freien Ende der Klemmarme allseits weich umhüllt werden, die Verletzungsgefahr des umgebenden Gewebes wird dadurch erheblich herabgesetzt.

Die nachfolgende Beschreibung bevorzugter Ausführungsformen der Erfindung dient im Zusammenhang mit der Zeichnung der näheren Erläuterung. Es zeigen:
- Figur 1:: eine Seitenansicht eines chirurgischen Nahtclips mit einem Überzug in Form eines Silikonschlauches im offenen Zustand;
- Figur 2:: eine Ansicht des Nahtclips der Figur 1 von den freien Enden her und
- Figur 3:: eine Ansicht des Nahtclips der Figuren 1 und 2 nach dem Anlegen an den Enden eines chirurgischen Nähfadens.

Der in der Zeichnung dargestellte chirurgische Nahtclip 1 umfaßt ein längliches, drahtähnliches Bauteil 2 aus Metall, beispielsweise aus Titan, welches einen etwa dreieckförmigen Querschnitt aufweist. Dieses Bauteil 2 ist so gebogen, daß zwei parallele, geradlinige Klemmarme 3, 4 einander gegenüberliegen, die beide über eine etwa 45°-Abwinklung 5, 6 in geradlinige Abschnitte 7, 8 eines Steges 9 übergehen, der die beiden Klemmarme 3, 4 miteinander verbindet. Die Abschnitte 7, 8 treffen sich dabei in der Mitte 10 des Steges 9 unter einem Winkel von etwa 90°. Die beiden Klemmarme 3, 4 liegen mit ihren ebenen Innenflächen 11, 12 somit im Abstand einander gegenüber und ermöglichen zwischen sich das Einführen eines festzuklemmenden Fadenendes eines chirurgischen Nähmaterials.

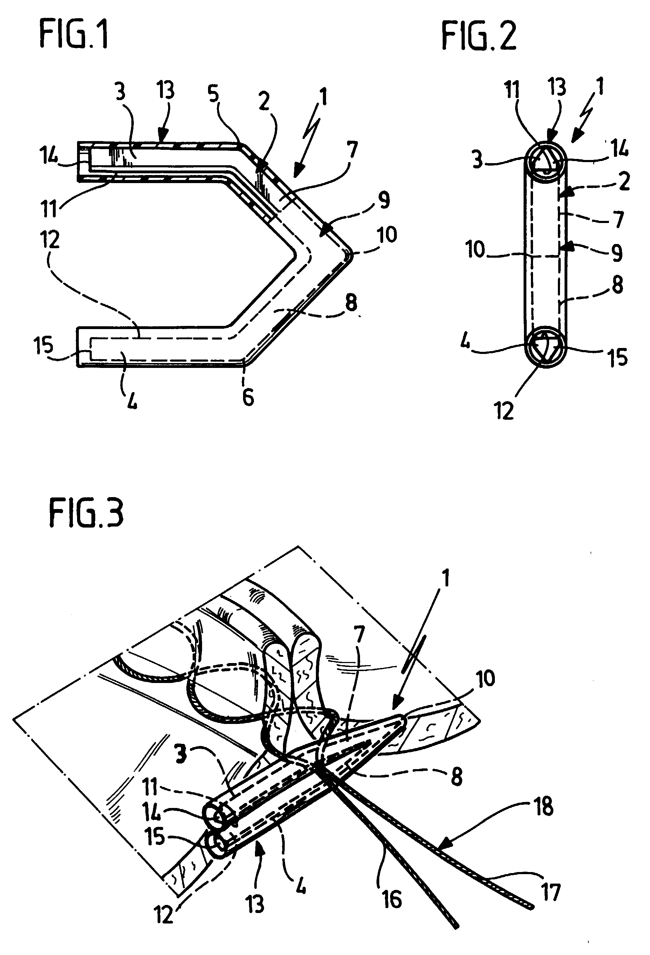

Über das gesamte Bauteil 2 ist ein schlauchförmiger Überzug 13 aus Silikon gezogen, und zwar derart, daß der Überzug 13 an den freien Enden 14, 15 der Klemmarme 3, 4 geringfügig über diese übersteht (Figur 1).

Bei einem bevorzugten Ausführungsbeispiel beträgt die Länge der Klemmarme etwa 3 bis 4 mm, die Länge der Abschnitte 7, 8 liegt bei etwa 2 bis 3 mm, der Außendurchmesser des schlauchförmigen Überzugs 13 bei etwa 1 mm. Bei anderen Ausführungsformen können diese Abmessungen abweichen, in jedem Falle handelt es sich bei diesen Nahtclips 1 jedoch um relativ kleine Bauteile, so daß es ohne weiteres möglich ist, diese mit geeigneten Instrumenten, beispielsweise mit Anlegezangen, auch bei endoskopischen Operationen durch enge Zugänge in den Körper einzuführen und dort zu applizieren.

Die beschriebenen chirurgischen Nahtclips 1 dienen dazu, die freien Enden 16, 17 von Nähfäden 18 relativ zueinander festzulegen, so daß eine Verknotung dieser freien Enden 16, 17 entfallen kann. Dies erfolgt dadurch, daß die offenen Nahtclips 1 (Figur 1) seitlich so an den Nähfaden 18 herangeführt werden, daß die freien Enden 16, 17 zwischen den Klemmarmen 3, 6 positioniert werden. Durch ein geeignetes, z.B. zangenförmiges Instrument, das an der Außenseite der Klemmarme 3, 4 anliegt, werden die Klemmarme 3, 4 gegeneinander verschoben, dabei verbiegt sich der Steg 9 insbesondere im Bereich der Abwinklungen 5, 6 und im Bereich der abgewinkelten Mitte 10, bis die Klemmarme 3, 4 gegeneinandergespannt sind und dadurch die freien Enden 16, 17 des Nähfadens 18 relativ zueinander festlegen (Figur 3). Die Verformung des Steges 9 ist dabei dauerhaft, d.h. nach dem Loslassen des Nahtclips 1 bleiben die Klemmarme 3, 4 allein aufgrund der Eigenstabilität des Steges 9 gegeneinandergespannt, Rastrücksprünge und dergleichen zum Schließen der Klemmarme 3, 4 sind nicht notwendig. Es ist für den Operateur dadurch möglich, durch behutsames Spannen der Klemmarme 3, 4 gegeneinander deren Endposition entsprechend der Stärke des Nähfadens 18 so zu wählen, daß einerseits die freien Enden 16, 17 des Nähfadens 18 sicher festgelegt sind und daß andererseits eine Beschädigung des empfindlichen Nahtgutes vermieden wird.

Die Fixierung der freien Enden 16, 17 des Nähfadens 18 wird durch das Anschmiegen des schlauchförmigen Überzuges 13 an die freien Enden 16, 17 unterstützt, gleichzeitig schützt dieser Überzug 13 den Nähfaden 18 gegen das Eindrücken scharfer Kanten des Bauteils 2, so daß dadurch eine Vergleichmäßigung des Anpreßdruckes eintritt und damit eine Herabsetzung der Beschädigungsgefahr.

Der in dieser Weise ausgeformte Nahtclip 1 kann rasch angelegt werden und wird aufgrund der Eigenstabilität des Bauteils 2 sicher in der Anlageposition gehalten, so daß ein Abgleiten und ein unbeabsichtigtes Lösen des Nähfadens im Bereich der freien Enden 16, 17 vermieden wird.

Günstig ist es auch, daß der Nahtclip 1 an den freien Enden 16, 17 des Nähfadens 18 zunächst mit geringer Spannung angelegt werden kann, so daß der Nahtclip 1 noch längs des Nähfadens 18 in die gewünschte Position verschoben werden kann, erst bei Erreichen der Endposition werden die Klemmarme 3, 4 dann kräftig gegeneinandergespannt, so daß sie längs der freien Enden 16, 17 des Nähfadens 18 festgelegt sind. Dies erleichtert insbesondere das Festlegen der freien Enden 16, 17 des Nähfadens 18 unter der gewünschten Spannung, da vor dem endgültigen Festklemmen des Nahtclips 1 der Chirurg die freien Enden 16, 17 des Nähfadens 18 fassen und diesen spannen kann, erst dann wird der Nahtclip 1 endgültig verklemmt und hält die einmal aufgebaute Spannung des Nähfadens 18.

## Patentansprüche

1. Chirurgischer Nahtclip (1) mit zwei gegeneinander verschwenkbaren Klemmarmen (3, 4), die im gegeneinandergeklemmten Zustand mindestens einen Nahtfaden (18) zwischen sich festlegen, dadurch gekennzeichnet, daß die Klemmarme (3, 4) über einen verformbaren und nach Verformung seine Form beibehaltenden Steg (9) miteinander verbunden sind.

2. Nahtclip nach Anspruch 1, dadurch gekennzeichnet, daß der Steg (9) aus Metall besteht.

3. Nahtclip nach Anspruch 2, dadurch gekennzeichnet, daß der Steg (9) und die Klemmarme (3, 4) aus einem durchgehenden Bauteil (2) aus Metall bestehen.

4. Nahtclip nach einem der Ansprüche 2 oder 3, dadurch gekennzeichnet, daß der Steg (9) und gegebenenfalls die Klemmarme (3, 4) aus Titan oder aus einer Titanlegierung bestehen.

5. Nahtclip nach einem der voranstehenden Ansprüche, dadurch gekennzeichnet, daß der Steg (9) V-förmig ausgebildet ist.

6. Nahtclip nach einem der voranstehenden Ansprüche, dadurch gekennzeichnet, daß die Klemmarme (3, 4) geradlinig ausgebildet sind und unter Ausbildung eines Winkels oder einer Abbiegung (5, 6) in den Steg (9) übergehen.

7. Nahtclip nach einem der voranstehenden Ansprüche, dadurch gekennzeichnet, daß er zumindest im Bereich der Klemmarme (3, 4) von einem weichen Material (13) umgeben ist.

8. Nahtclip nach Anspruch 7, dadurch gekennzeichnet, daß das Material (13) Silikon ist.

9. Nahtclip nach Anspruch 7 oder 8, dadurch gekennzeichnet, daß zumindest die Klemmarme (3, 4) und gegebenenfalls der gesamte Nahtclip (1) einen Tauchüberzug aus dem weichen Material (13) tragen.

10. Nahtclip nach Anspruch 7 oder 8, dadurch gekennzeichnet, daß zumindest die Klemmarme (3, 4) und gegebenenfalls der gesamte Nahtclip (1) einen Spritzüberzug aus dem weichen Material (13) tragen.

11. Nahtclip nach einem der Ansprüche 7 oder 8, dadurch gekennzeichnet, daß das weiche Material (13) die Form eines Schlauches aufweist, der zumindest über die Klemmarme (3, 4) und gegebenenfalls über den gesamten Nahtclip (1) gezogen ist.

12. Nahtclip nach einem der Ansprüche 7 bis 11, dadurch gekennzeichnet, daß das weiche Material (13) über die freien Enden (14, 15) der Klemmarme (3, 4) übersteht.
